(19) Europäisches Patentamt
European Patent Office
Office européen des brevets

(11) Publication number: **0 167 179**
**A1**

# (12) EUROPEAN PATENT APPLICATION

(21) Application number: 85109170.2

(22) Date of filing: 30.08.83

(51) Int. Cl.[4]: C 07 C 121/40
C 07 C 121/76, C 07 C 121/413

(30) Priority: 31.08.82 JP 151003/82
31.08.82 JP 151004/82

(43) Date of publication of application:
08.01.86 Bulletin 86/2

(84) Designated Contracting States:
CH DE FR GB LI

(60) Publication number of the earlier application in accordance with Art. 76 EPC: 0 102 086

(71) Applicant: Daikin Kogyo Co., Ltd.
Shinhankyu Building No 1-12-39, Umeda Kita-ku
Osaka-shi Osaka-fu(JP)

(72) Inventor: Ishikawa, Nobuo, Dr.
10-10, Shinoharadai-machi Kohoku-ku
Yohokama-shi Kanagawa-ken(JP)

(72) Inventor: Takaoka, Akio, Dr.
17-109, Dobashi 1-chome Takatsu-ku
Kawasaki-shi Kanagawa-ken(JP)

(74) Representative: Patentanwälte TER MEER - MÜLLER - STEINMEISTER
Mauerkircherstrasse 45
D-8000 München 80(DE)

## (54) Alpha-substituted-alpha-fluoro-alpha-cyanoacetic acids and their derivatives.

(57) α-substituted-α-fluoro-α-cyanoacetic acids and their derivatives are disclosed that are expressed by the general formula:

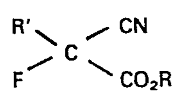
,

wherein R is an aliphatic or aromatic hydrocarbon group, or hydrogen or alkali metal atom, and R' is a group as derived from an unsaturated compound expressed by the following general formula:

$$YCH = CHR'' \quad \text{or} \quad YC \equiv CR'',$$

wherein R" is a hydrogen atom or aliphatic group and Y is a highly electronegative group with or without formation of a ring by bonding to a carbon atom to which the group R" is bonded, and which can be used as agricultural chemicals or as intermediates for their manufacture.

EP 0 167 179 A1

## BACKGROUND OF THE INVENTION

### Title of the Invention

α-substituted-α-fluoro-α-cyanoacetic acids and their derivatives

### Field of the Invention

This invention relates to α-substituted-α-fluoro-α-cyanoacetic acids and their derivatives.

### Description of the Priot Art

There are known many monofluoro compounds that have physiological activities and/or are efficacious as agricultural chemicals or medicines. As a building block of such monofluoro compounds, the α-fluoro-α-cyanoacetic acid esters are thus considered to be very important intermediates.

## OBJECTS AND SUMMARY OF THE INVENTION

Accordingly, it is an object of the present invention to provide new and useful fluorine containing cyanoacetic acids and their derivatives.

Namely, the invention provides α-substituted-α-fluoro-α-cyanoacetic acids and their derivatives that are expressed by the following general formula:

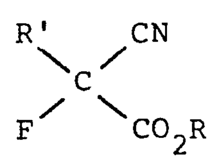 ,

wherein R is an aliphatic or aromatic hydrocarbon group,

or hydrogen or alkali metal atom and R' is a group that derived from an unsaturated compound expressed by the following general formula:

$$YCH = CHR" \text{ or } YC \equiv CR",$$

wherein R" is a hydrogen atom or aliphatic group preferably having carbon atoms of 1 to 6, and Y is a highly electronegative group with or without formation of a ring with the carbon atom to which R" is bonded.

The above cyanoacetic acids and their derivatives of the invention have a fluorine atom in their molecular structure and therefore exhibit superior properties of the fluoro compound while their cyano group gives them an efficacy as agricultural chemicals such as herbicides.

R in the above general formula may preferably be selected from among alkyl groups having up to 10 carbon atoms, for example, methyl, ethyl, propyl and butyl groups, and allyl and other alkenyl groups, aryl and other unsubstituted and substituted aromatic hydrocarbon groups.

Y is preferably an alkylcarbonyl or alkoxycarbonyl group preferably having carbon atoms of 1 to 6, or cyano group bonded to a carbon atom comprising the unsaturated carbon-carbon bond.

DESCRIPTION OF THE PREFERRED EMBODIMENTS

Examples of the compounds of the present invention will be concretely described in terms of their manufacturing method.

For example, using hexafluoropropane (HFP) as the starting material, the following reaction is conducted to prepare ethyl α-fluoro-α-cyanoacetate 2 through tetrafluoropropionitrile 1 with ethyl monofluoromalonate 3 as by-product:

$$CF_3CF{=}CF_2 \xrightarrow[0\text{-}10^\circ C]{NH_3\ aq./Dioxane} CF_3CHFCN$$

(Bubbling) (Yield: 75-80%)

$$1) \xrightarrow{1)\ NaOH/C_2H_5OH \quad 2)\ H_3O^+} NCCHFCO_2C_2H_5$$

2

(Yield: 60%)

+

$C_2H_5O_2CCHFCO_2C_2H_5$

(Yield: 25%)

In the above reaction, the nitrile 1 is produced in a short reaction time with a much improved yield by using a dry ice-acetone condenser and having HFP bubbled into an ammonia-dioxane sulution at a controlled reaction temperature. The above synthesis of compound 1 is conducted by a method as disclosed by Knunyants and others wherein ammonia, if used in an excessive amount and particularly more than several times its stoichiometric quantity, a much improved yield to 75 to 80% as mentioned above can be obtained.

Next, this nitrile 1 is subjected to alcoholysis by reacting with an alkoxide or alcoholic potassium hydroxide solution. The reaction mixture is then acidified by addition of hydrochloric acid or the like for the selective formation of the above malonic ester 3. If, after the alcoholysis under the action of an alcoholic alkali hydroxide solution, for example, ethanolic or propanolic sodium hydroxide solution, the alcohol is removed and hydrochloric acid is then added for

acidification, the product 2 will be produced almost in a yield of 70%.

Since the products 2 and 3 have boiling points that differ by 35°C, it is possible to isolate and purify the compound 2 by rectification (yield of 2: 60%, boiling point: 174-175°C).

A possible reaction scheme for the mechanism by which the compound 2 is formed from 1 is given below, in which the reaction proceeds through formation of perfluoroacrylonitrile as an intermediate. It is assumed that ethyl fluorocyanoorthoacetate 4 is then formed. If it is hydrolyzed by an acid, such as HCl, $H_2SO_4$ or the like, there is produced the compound 2. Further, partially, the hydrochloride of ethyl α-fluorocarboethoxyimidoacetate is formed and, as its cyano group is decomposed, the compound 3 is produced.

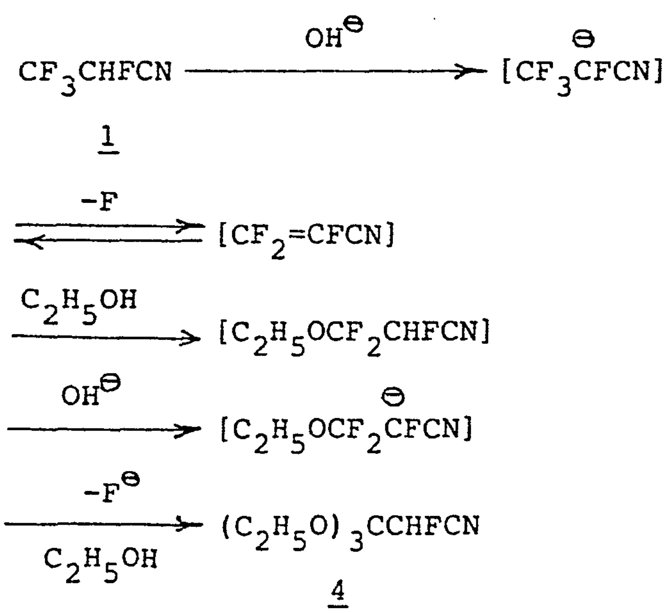


1

4

$$\xrightarrow{H_3O^+} C_2H_5O_2CCHFCN$$

2

+

$$C_2H_5O_2CCHFCO_2C_2H_5$$

3

It is noted that the ester 2 produced from the above reaction can be hydrolyzed to α-fluoro -α-cyanoacetic acid 5 or treated with alkali hydroxide to form an alkali salt 6, for example, sodium salt of α-fluoro-α-cyanoacetic acid according to the following reaction:

$$C_2H_5O_2CCHFCN \xrightarrow{H^+} HO_2CCHFCN + C_2H_5OH$$

5

or $$C_2H_5O_2CCHFCN \xrightarrow{NaOH} NaO_2CCHFCN + C_2H_5OH$$

6

In the above method of manufacturing the compound 2, HFP industrially produced is used as the starting material and it is reacted with ammonia and the resultant compound is particularly subjected to an alcoholysis reaction followed by a treatment with a mineral acid. So the α-fluoro-α-cyanoacetate ester can be produced at a low cost under a harmless condition.

The alkyl (ethyl) group in the ethoxycarbonyl group of

the above ester 2 derives from the alkoxide or alcohol used in the alcoholysis as mentioned above.

If the hydrocarbon group of such alkoxide or alcohol is selected so as to correspond to the given R, therefore, the alkylated or alkenylated ester of the invention can be produced as mentioned below.

For example, the above ethyl α-fluoro-α-cyanoacetate can be alkylated or alkenylated under presence of various bases at room temperature. Since, among these bases, NaH performs excellently, first, the ester is added to ice-cooled NaH in diglyme to form a carbanion from the compound 2 , then, an alkyl or alkenyl halide R'X where R' is particularly an aliphatic hydrocarbon group having up to 10 carbon atoms and X is a halogen atom is added dropwise for agitation at room temperature. The above reaction of alkylation or alkenylation proceeds according to the following formula, which gives the alkylated or alkenylated products as expressed by the chemical formula 7 in high yield as in Table 1:

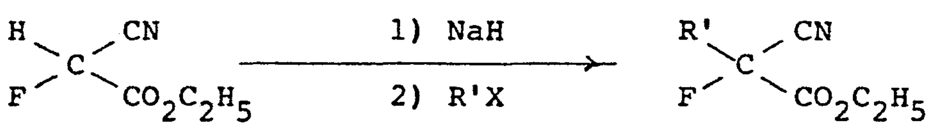


2 7

Table 1

| R'X | Reaction time, hr | R' of product 7 | $^{19}$FNMR Yield, % | $^{19}$FNMR $\delta$(ppm) | $J_{HF}$ $(H_2)$ |
|---|---|---|---|---|---|
| $CH_3Br$ | 2 | $CH_3$- | 86 | 71.0 (q) | 21.8 |
| $C_2H_5Br$ | 5 | $C_2H_5$- | 73 | 78.5 (t) | 20.7 |
| $CH_2=CHCH_2Br$ | 5 | $CH_2=CHCH_2$- | 71 | 81.0 (t) | 21.4 |
| $n-C_4H_9Br$ | 5 | $n-C_4H_9$- | 74 | 114.6 (t) | 21.6 |

Having a cyano group, such alkylated or alkenylated $\alpha$-fluoro-$\alpha$-cyanoacetate ester 7 is superbly efficacious as agricultural chemicals, for example, as a herbicide. In addition, the above compounds 2 and 7 can be used to synthesize derivatives of pyrimidine.

Thereafter, the above compound 2 is subjected to a 10 min reaction under agitation and under presence of a catalytic amount of spray-dried KF in sulfolane at room temperature to produce the following carbanion 8:

$NCCHFCO_2C_2H_5$

2

$$\xrightarrow{\text{Base}} [NCC^{\ominus}FCOO-C_2H_5]$$

8

Various acceptors of the Michael reaction or Michael acceptors can then be added to the above carbanion in an ice-cooled condition to give a reaction as expressed by the following formula at room temperature for immediate

conversion to the corresponding Michael adducts at a high yield:

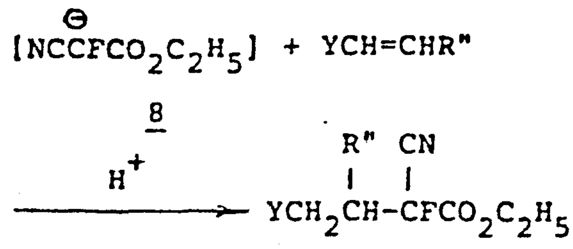


8

9

wherein YCH=CHR" is a Michael acceptor, R" being a hydrogen atom or alkyl group and Y a highly electronegative group, for example, alkylcarbonyl, alkoxycarbonyl or cyano group. Y may form a ring by bonding to the carbon atom to which R" is bonded. The above Michael reaction can also use an acceptor that has a carbon-carbon triple bond. In this case, the reaction proceeds as follows:

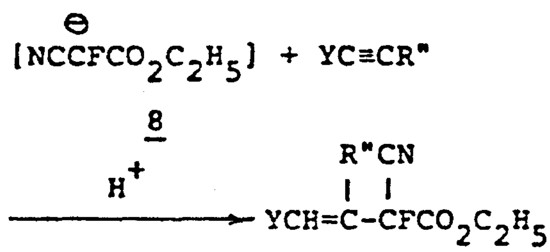


8

9

The following Table 2 gives several examples of the Michael adducts 9 as derived from the ethyl α-fluoro-α-cyanoacetate by the above method.

Table 2

| Michael acceptor | Reaction time | Product (Michael adduct) | $^{19}F$ NMR Yield, % | $^{19}F$ NMR δ(ppm) | $^{19}F$ NMR $J_{HF}(H_2)$ |
|---|---|---|---|---|---|
| 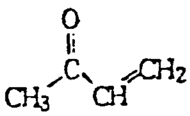 | 10 min | 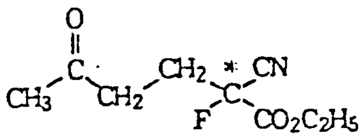 | 79 | 77.3 (t) | 20.9 |
| 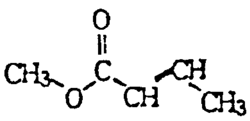 | 2 hr | 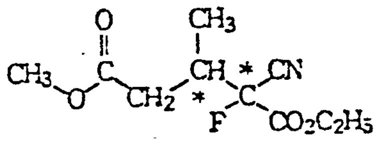 | 95 | 81.3 (d)<br>82.8 (d) | 16.9 |
| 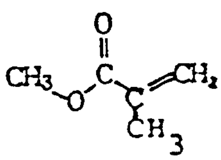 | 2 hr | 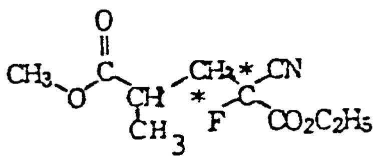 | 94 | 76.5(dd)<br>78.8(dd) | 19.8<br>21.6 |
| 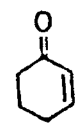 | 10 min | 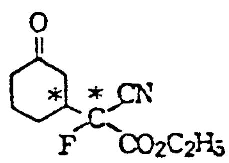 | 99 | 83.5 (d)<br>84.5 (d) | 17.9 |
| 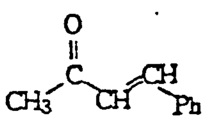 | 10 min | 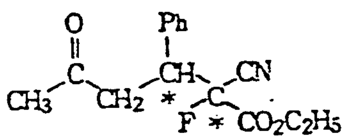 | 93 | 78.9 (d)<br>81.4 (d) | 22.0<br>22.9 |
| 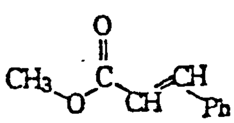 | 15 hr | 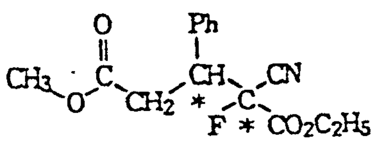 | 92 | 79.5 (d)<br>83.5 (d) | 20.7<br>23.5 |

These Michael adducts can be used as agricultural chemicals, for example, herbicide, insecticide or these intermediates.

The above embodiments are set forth as a further description but are not to be construed as limiting the invention thereto. Modifications and variations are

possible without departing from the spirit and scope of the invention.

For example, if alkylated or alkenylated as mentioned above, the foregoing compound 5 or 6 gives the corresponding alkylated or alkenylated $\alpha$-fluoro-$\alpha$-cyanoacetic acids or their alkali metal salts.

When subjected to the Michael reaction as mentioned above, the same compound 5 or 6 gives the corresponding Michael adduct of $\alpha$-fluoro-$\alpha$-cyanoacetic acid or its alkali metal salt.

The invention will be understood more clearly with reference to the following Examples:

Manufacturing Example

In a three-necked flask equipped with an efficient dry-ice condenser, 1000 g of conc. ammonia water and 500 ml of dioxane are placed. Into this mixture, 291 g of hexafluoro propene gas was introduced at the rate of 0.9 g/min, keeping the temperature at $0\pm2$ °c . After the introduction, the reaction mixture was stirred for 2 hrs at -5 to 0 °c, extracted with xylene, washed with water, dried over magnesium sulfate and subjected to distillation.

Thus 183 g of 2,3,3,3-tetrafluoropropio-nitrile, b.p. 39-40 °c, was obtained in a 74 % yield.

Instead of 1,4-dioxane in the preceding procedure, the same amount of tetrahydrofuran can be used.

In a similar manner 295 g of hexafluoropropene used giving 206 g (82 % yield) of 2,3,3,3-tetrafluoropropionitrile.

In a 2 ℓ-flask a solution of 120 g (3 mol) of sodium hydroxide in 1 ℓ of ethanol was placed and 127 g (1 mol) of 2,3,3,3-tetrafluoropropionitrile was added dropwise at room temperature and stirred for 1 hr. From the reaction mixture 500 ml of ethanol distilled out and the residue was poured into 1 ℓ of water. The oily material was extracted with chloroform, washed with water 5 times, and dried over calcium chloride.

To the extract thus obtained 80 ml of conc. hydrochloric acid was added and the mixture was stirred at room temperature for 1h. After neutralization with a saturated aqueous solution of sodium bicarbonate, an oily layer was separated, washed with water and dried over magnesium sulfate. On removing chloroform 79 g (60 %) of ethyl α-fluoro-α-cyanoacetate and 46 g (26 %) of ethyl fluoromalonate were distilled out at 174-175 °C and 208-210 °C, respectively. The product, that is, ethyl α-fluoro-α-cyanoacetate showed the following properties:

| | |
|---|---|
| IR(neat): | 2260 (CN), 1780,1760 $cm^{-1}$ (Ester) |
| $^{19}F$ NMR($CHCl_3$): | $\delta$ 114.0 (d) ($J_{HF}$=45.5 Hz) |
| $^{1}H$ NMR($CDCl_3$): | $\delta$ 5.47 (d) ($J_{HF}$=47.4 Hz), 4.30(q), 1.30 (t) |
| MS (m/e): | 131 ($M^+$) |

Example 1

Into a mixture of 290 mg (5 mmol) of spray-dried potassium fluoride and 10 ml of sulfolane, 2.65 g (20 mmol) of ethyl α-fluoro-α-cyano acetate as obtained above was added dropwise at room temperature. After agitation of the mixture for 10 minutes, 2.92 g (20 mmol) of benzal acetone was added dropwise under ice cooling to the reaction solution, then stirred for 10 minutes at 0 to 5 °C. Thereafter, a saturated ammonium chloride solution was added to the mixture, an oily material was extracted with ether and dried over magnesium sulfate. After the ether was distilled out 4.7 g of a Michael adduct of b.p. 141 to 142 °C/2 mmHg was obtained in a yield of 85 % by a destillation under reduced pressure. This product showed the following analytical properties:

IR (neat): 2260 (CN), 1715 (CO), 1770 $cm^{-1}$ (Ester)

$^{19}F$ NMR ($CCl_4$): $\delta$ 78.9 (d, $J_{HF}$=22.0 Hz), 81.4 (d, $J_{HF}$ =22.9 Hz)

$^{1}H$ NMR ($CCl_4$): $\delta$ 1.00-1.30 (2t), 2.00 (s), 3.05 to 3.21 (2d), 3.80-4.32 (2q+m), 7.22-7.48 (m)

MS (m/e): 277 ($M^+$)

Example 2

Into a mixture of 290 mg (5 mmol) of spray-dried potassium fluoride and 10 ml of sulfolane, 2.65 g (20 mmol)

of ethyl α-fluoro-α-cyanoacetate was added dropwise at room temperature. After agitation for 10 minutes at 0 to 5°C, 3.25 g (20 mmol) of methyl cinnamate was added dropwise under ice cooling to the reaction mixture and these were reacted for 15 hours at room temperature. Then, the reaction mixture was treated similarly to Example 1 to provide 4.8 g of a Michael adduct of b.p. 134-135 °C/3 mmHg in a yield of 83 % through a reduced-pressure distillation. This product had the following properties:

| | |
|---|---|
| IR (neat): | 2260 (CN), 1770, 1745 $cm^{-1}$ (Ester) |
| $^{19}F$ NMR ($CCl_4$): | $\delta$ 79.5 (d, $J_{HF}$=20.7 Hz), |
| | 83.5 (d, $J_{HF}$=23.5 Hz) |
| MS (m/e): | 293 ($M^+$) |

C L A I M S

1. α-substituted-α-fluoro-α-cyanoacetic acids and their derivatives that are expressed by the general formula:

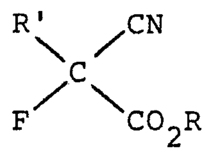 ,

wherein R is an aliphatic or aromatic hydrocarbon group or hydrogen or alkali metal atom, and R' is a group as derived from an unsaturated compound expressed by the following general formula:

$$YCH = CHR'' \quad \text{or} \quad YC \equiv CR'' ,$$

wherein R" is a hydrogen atom or aliphatic group and Y is a highly electronegative group with or without formation of a ring by bonding to a carbon atom to which the group R" is bonded.

2. The α-substituted-α-fluoro-α-cyanoacetic acids and their derivatives as claimed in claim 1, c h a r a c t e r i z e d in that R is an alkyl or alkenyl group having up to 10 carbon atoms, or an aryl group.

3. The α-substituted-α-fluoro-α-cyanoacetic acids and their derivatives as claimed in claim 1, c h a r a c t e r i z e d in that the group Y is an alkylcarbonyl or alkoxycarbonyl or cyano group bonded to a carbon atom comprising the unsaturated carbon-carbon bond.

European Patent Office

# PARTIAL EUROPEAN SEARCH REPORT

which under Rule 45 of the European Patent Convention shall be considered, for the purposes of subsequent proceedings, as the European search report

0167179

Application number

EP 85 10 9170

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF MEDICINAL CHEMISTRY, vol. 10, no.4, July 26, 1967, pages 536-541; American Chemical Society, US H. GERSHON et al.: " Organic fluorine compounds. III. Action of perchloryl fluoride on substituted ethyl cyanoacetates and animal toxicities of the fluorinated products1-3." | | C 07 C 121/40<br>C 07 C 121/76<br>C 07 C 121/413 |
| | *Page 537, table II, compound Vj* | 1-3 | |
| | ------- | | |

TECHNICAL FIELDS SEARCHED (Int. Cl.4)

C 07 C 121/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 3

Claims searched incompletely: 1,2

Claims not searched:

Reason for the limitation of the search: The definition of Y: "A highly electronegative group" is ambiguous.

| Place of search | Date of completion of the search | Examiner |
|---|---|---|
| The Hague | 11-10-1985 | THEUNS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82